# EUROPÄISCHE PATENTSCHRIFT

(11) **EP 0 779 506 B1**
(45) Veröffentlichungstag und Bekanntmachung des Hinweises auf die Patenterteilung: **07.11.2001**
(21) Anmeldenummer: 96108508.1
(22) Anmeldetag: 29.05.1996
(51) Int. Cl.: G01N 13/00, G01N 33/15, G01N 33/18

(54) **Dissolutionstestgerät**
Dissolution testing device
Dispositif d'essai de dissolution

(30) Priorität: 12.12.1995 DE 29519713 U
(43) Veröffentlichungstag der Anmeldung: 18.06.1997
(73) Patentinhaber: ERWEKA GmbH, D-63150 Heusenstamm (DE)
(72) Erfinder: Müller, Werner G., D-63150 Huesenstamm (DE)
(74) Vertreter: Kirschner, Klaus Dieter, Dipl.-Phys.

(56) Entgegenhaltungen:
- EP-A- 0 204 343
- EP-A- 0 343 261
- EP-A- 0 635 713
- US-A- 4 594 902
- US-A- 5 076 107

## Beschreibung

Die Erfindung bezieht sich auf ein Dissolutionstestgerät mit mehreren Prüfbehältern zur Aufnahme von Dissolutionsproben, mehreren Rührelementen zum Rühren der Dissolutionsprobe, die in einem höhenverstellbaren Gehäuse angeordnet sind, einer Entnahmeeinrichtung mit Entnahmerohren zur Entnahme von vorgegebenen Volumenanteilen der Dissolutionsprobe aus den Prüfbehältern, die in Abhängigkeit des Füllstandes der Dissulationsprobe und der verwendeten Rührelemente an einer vorgegebenen Arbeitsposition in den Prüfbehältern anzuordnen sind, sowie eine Rechnereinrichtung zum Speichern von Meßdaten und vorgegebenen Parametern.

Bisher werden bei Geräten zum Testen von Dissolutionsproben die Entnahme und die Rückführung von Volumenanteilen der Dissolutionsproben mit Hilfe von einer Bedienperson durchgeführt. Diese Bedienperson ist beispielsweise für die geforderte Positionierung von Entnahmerohren einer Entnahmeeinrichtung in jedem Prüfbehälter eines Dissolutionstestgeräts zuständig, wobei die Positionierung der Entnahmerohre der Entnahmeeinrichtung in Abhängigkeit von der Art des Rührwerkzeuges und dem Flüssigkeitsstand der Dissolutionsprobe abhängt. Die geforderte Arbeitsposition des Entnahmerohres liegt auf halber Höhe zwischen Oberkante des Rührorganes und der Linie des Füllstandes.

Dementsprechend ist eine Kontrolle der geforderten Arbeitsposition der Entnahmerohre der Entnahmeeinrichtung nur mit Hilfe einer Sichtkontrolle der Bedienperson möglich. Diese Sichtkontrolle ist unter anderem von Parametern, wie Sichtbarkeit des Prüfbehälters, durch die Außenwand des Prüfbehälters hervorgerufene Reflexfehler, sowie körperliche und geistige Verfassung der Bedienperson abhängig.

EP-A-0 204 343 offenbart ein Dissolutionstestgerät nach dem Oberbegriff des Anspruchs 1. Die Höhenverstellung der Rührer und der Entnahmerohre erfolgt über zwei völlig getrennte Mechanismen.

EP-A-0 343 261 offenbart ein weiteres Dissolutionstestgerät, bei dem die Verstellung der Entnahmerohre über zwei Teleskopsäulen erfolgt.

EP-A-0 635 713 offenbart die Verwendung eines Schrittmotors im Zusammenhang mit einem Dissolutionstestsystem, wobei der Schrittmotor verwendet werden, um die feste Tablette in den Prüfbehälter einzufüllen.

Die Aufgabe der vorliegenden Erfindung ist es, ein Dissolutionstestgerät bereitzustellen, das eine zuverlässige, exakte Positionierung der Entnahmerohre einer Entnahmeeinrichtung ermöglicht, die reproduzierbar ist und ein Versagen einer Bedienperson ausschließt.

Die Aufgabe wird durch ein Dissolutionstestgerät nach Anspruch 1 gelöst. Die abhängigen Ansprüche betreffen weitere vorteilhafte Aspekte der Erfindung.

Vorteilhafte Ausgestaltungen der Erfindung ergeben sich aus den Unteransprüchen.

Ein Ausführungsbeispiel der Erfindung wird nun anhand der beiliegenden Zeichnungen beschrieben. Es zeigen:
- Fig. 1:: in schematischer Darstellung ein Dissolutionstestgerät während der Justierung der Rührelemente;
- Fig. 2:: in schematischer Darstellung das Dissulotionstestgerät nach der Justierung, wobei die Rührelemente sich in einer Arbeitsposition befinden;
- Fig. 3:: in schematischer Darstellung eine Einrichtung zum Einführen der Entnahmerohre in die Testbehälter nach der Justierung der Rührelemente; und
- Fig. 4:: einen Teil der Entnahmeeinrichtung im Detail.

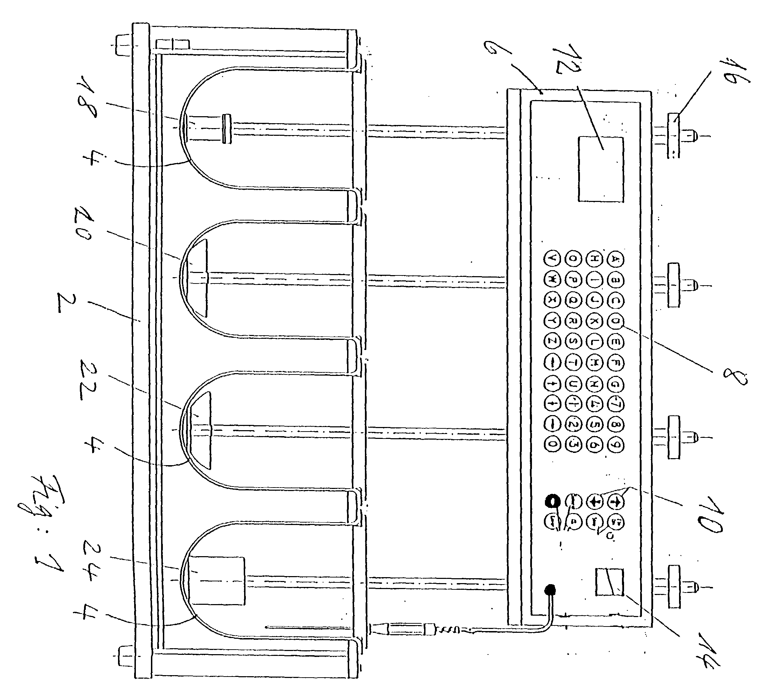

In Figur 1 ist schematisch ein Dissolutionstestgerät dargestellt. Das Dissolutionstestgerät weist ein Gestell 2 für Prüfbehälter 4 auf, die in zwei Reihen zu je vier Prüfbehältern angeordnet sind, wobei nur die vorderen vier Prüfbehältern schematisch dargestellt sind. Über dem Gestell 2 ist über Teleskopsäulen (nicht gezeigt) verstellbar ein Gehäuse 6 angeordnet, welches einen Rechner, das Bedienerfeld 8 für die Eingabe, das Bedienerfeld 10 für die Steuerung des Gerätes, ein Anzeigefeld 12, einen Netzstecker 14 sowie Klemmeinrichtungen 16 für die Halterung von Rührwerkzeugen 18, 20, 22, 24 aufweist.

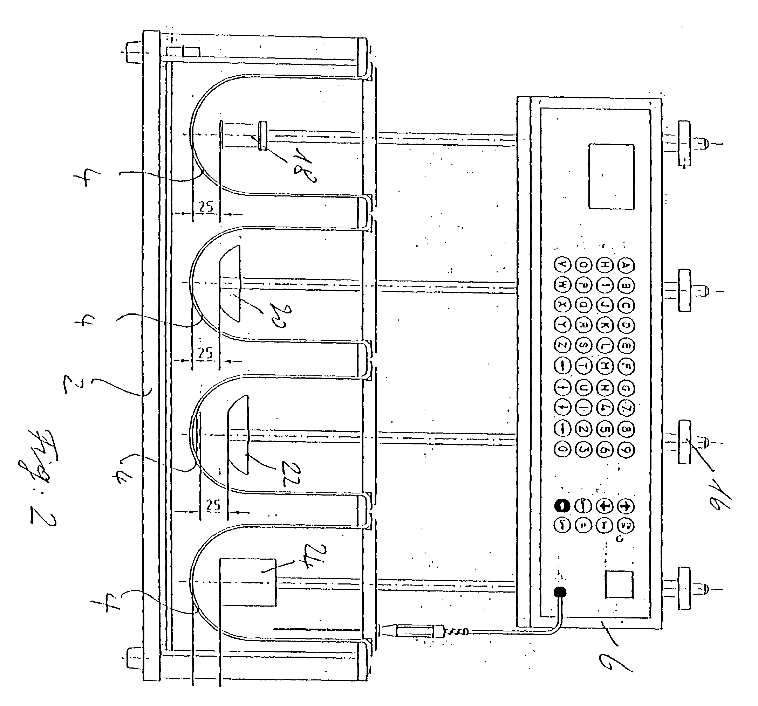

In Figur 1 sind nur zum Zwecke der Erläuterung unterschiedliche Rührwerkzeuge 18 bis 24 dargestellt, obwohl in der Praxis selbstverständlich nur ein Typ von Rührwerkzeugen für einen bestimmten Test verwendet wird. Die verschiedenen Arten von Rührwerkzeugen sind verschiedenen Testmethoden zugeordnet, wobei das Rührwerkzeug 18 ein Körbchen, das Rührwerkzeug 20 ein Paddel, das Rührwerkzeug 22 ein Paddel über einer Scheibe und das Rührwerkzeug 24 ein Transdermal-Zylinder ist. Bei der Justierung der Rührwerkzeuge werden diese zunächst in die in Figur 1 gezeigte Position nach unten gefahren, die praktisch die Nullposition ist. Danach werden die Rührwerkzeuge durch Verfahren des Gehäuses 6 auf einen Abstand von 25 +- 2mm von der Nullposition in die Arbeitsposition gebracht, die in Figur 2 dargestellt ist.

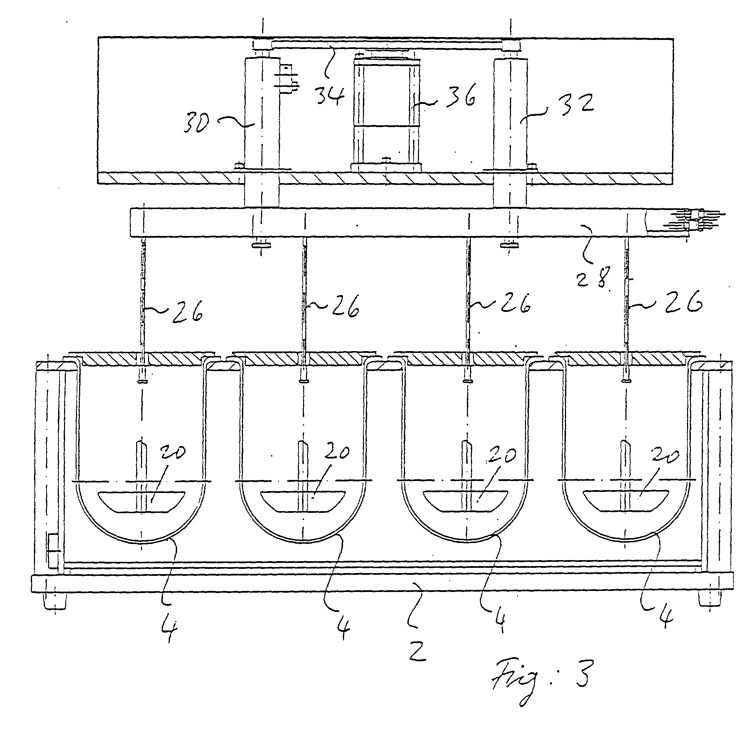

Die verschiedenen Testvorschriften erfordern, daß die Entnahmerohre mit ihrer Entnahmeöffnung auf die Hälfte zwischen der Oberkante des Rührwerkzeuges und der Prüfstandmarke der Probe in Position gebracht werden. In Figur 3 ist nun realistisch dargestellt, daß die Rührwerkzeuge 20 in Form von Paddeln einheitlich in allen Prüfbehältern angeordnet und in Position gebracht worden sind.

In Figur 3 ist ferner zusätzlich zu der Darstellung von den Figuren und 2, die Anordnung der Entnahmerohre 26 auf einem Rahmen 28 dargestellt, der über Teleskopsäulen 30, 32 höhenverstellbar an dem Gehäuse 6 angeordnet ist. Die Teleskopsäulen 30, 32 sind über einen Zahnriehmen 34 mit einem Schrittmotor 36 gekoppelt, der den Rahmen 28 und damit die Entnahmerohre 26 nach den Eingabedaten über den Füllstand und das verwendete Rührwerkzeug programmgesteuert in die jeweilige Arbeitsposition verfährt. Die entsprechenden Daten über das verwendete Rührwerkzeug wurden in den Rechner in dem Gehäuse 6 bei der Justierung der Rührwerkzeuge eingegeben, während die Daten über den Füllstand bei den jeweiligen Dissolutionsproben bei der Einstellung des Gerätes auf einen bestimmten Test eingegeben wurde. Die relevanten Daten stehen daher dem Rechner nach Verarbeitung durch das Programm zur Verfügung und könen an den Schrittmotor in Form von Schrittschaltimpulsen weitergegeben zu werden.

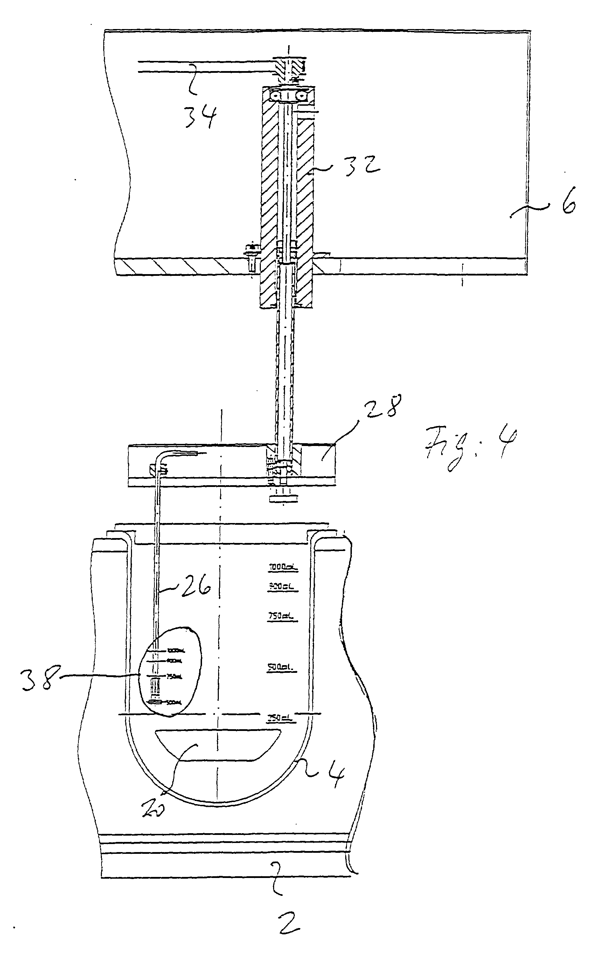

Figur 4 zeigt in einem größeren Detail die Relation zwischen den Füllständen 1.000 ml, 900 ml, 750 ml und 500 ml und den entsprechenden Arbeitspositionen der Entnahmerohre 26 die bei 38 ebenfalls mit 1.000 ml, 900 ml, 750 ml und 500 ml bezeichnet sind. Es ist zu beachten, daß die Arbeitspositionen der Entnahmerohre 26 je nach dem verwendeten Rührwerkzeug unterschiedlich sind. Daher sind die Figur 4 gezeigten Positionen nur für eine Prüfmethode, in der ein Paddel verwendet wird, zutreffend. Würde bei einer Prüfmethode statt dem Paddel ein Körbchen verwendet, würden sich andere Arbeitspositionen für die Entnahmerohre ergeben. Daraus ist ersichtlich, daß es Probleme gibt, wenn diese Arbeitspositionen nicht, wie bei der vorliegenden Ausführungsform, programmgesteuert angefahren werden. Man müsste dann für jede Prüfmethode entweder besonders geeichte Prüfbehälter, in denen die Arbeitspositionen der Entnahmerohre markiert sind, verwenden oder man müsste an einem Behälter mehrere Markierungen für die unterschiedlichen Rührwerkzeuge anbringen. Beides ist jedoch nicht genügend sicher, weil zu unübersichtlich.

## Patentansprüche

1. Dissolutionstestgerät mit mehreren Prüfbehältern zur Aufnahme von Dissolutionsproben, mehreren Rührelementen zum Rühren der Dissolutionsprobe, die in einem höhenverstellbaren Gehäuse angeordnet sind, einer Entnahmeeinrichtung mit Entnahmerohren zur Entnahme von vorgegebenen Volumenanteilen der Dissolutionsprobe aus den Prüfbehältern, die in Abhängigkeit des Füllstandes der Dissolutionsprobe und der verwendeten Rührelemente an einer vorgegebenen Arbeitsposition in den Prüfbehältern anzuordnen sind, wobei die Entnahmerohre (26) auf einem höhenverstellbaren Rahmen (28) angeordnet sind, sowie eine Rechnereinrichtung zum Speichern von Meßdaten und vorgegebenen Parametern, **dadurch gekennzeichnet, daß** der Rahmen (28) an dem Gehäuse (6) höhenverstellbar angeordnet ist, und daß ein Motor (36) in dem Gehäuse vorgesehen ist, der den Rahmen (28) und damit die Entnahmerohre (26) nach den Eingabedaten über den Füllstand und das Rührelement programmgesteuert in die jeweilige Arbeitsposition verfährt.

2. Dissolutionstestgerät nach Anspruch 1, **dadurch gekennzeichnet, daß** der Motor (36) ein Schrittmotor ist.

3. Dissolutionstestgerät nach Anspruch 1, **dadurch gekennzeichnet, daß** der Motor (36) über einen Zahnriemen (34) mit zwei Teleskopsäulen (30, 32) gekoppelt ist, die den Rahmen tragen.

## Claims

1. Solution testing device including a plurality of test containers for receiving solution samples, a plurality of stirring elements for stirring the solution samples, which are arranged in a vertically movable housing, a removal device with removal tubes for removing predetermined amounts of the solution samples from the test containers, which should be arranged at a predetermined working position in the test containers in dependence on the filling level of the solution samples and the stirring elements used, and a computer device for storing measured data and predetermined parameters, **characterised in that** the removal tubes (26) are arranged on a frame (28), that the frame (28) is vertically movably arranged on the housing (6) and that a motor (36) is provided, which moves the frame (28) and thus the removal tubes (6) into the respective working position in a program controlled manner in accordance with the input data relating to the filling level and the stirring element.

2. Solution testing device as claimed in claim 1, **characterised in that** the motor (36) is a stepper motor.

3. Solution testing device as claimed in claim 1, **characterised in that** the motor (36) is coupled via a toothed belt (34) to two telescopic columns (30,32), which carry the frame.

## Revendications

1. Appareil de test de dissolution comportant plusieurs réceptacles d'essai pour recevoir des échantillons de dissolution, plusieurs éléments agitateurs pour agiter les échantillons de dissolution, qui sont agencés dans un boîtier réglable en hauteur, un dispositif de prélèvement comprenant des tubes de prélèvement pour prélever des proportions volumiques prédéterminées de l'échantillon de dissolution hors des réceptacles d'essai que l'on doit agencer à une position de travail prédéterminée dans les réceptacles d'essai en fonction du niveau de remplissage de l'échantillon de dissolution et des éléments agitateurs utilisés, les tubes de prélèvement (26) étant agencés sur un cadre (28) réglable en hauteur, ainsi qu'un dispositif de calcul pour mémoriser des données de mesure et des paramètres donnés, **caractérisé en ce que** le cadre (28) est agencé de façon réglable en hauteur sur le boîtier (6), et **en ce qu'**il est prévu un moteur (36) dans le boîtier, qui déplace de façon commandée par programme le cadre (28) et ainsi les tubes de prélèvement (26) jusque dans la position de travail respective en fonction des données entrées concernant le niveau de remplissage et sur l'élément agitateur.

2. Appareil de test de dissolution selon la revendication 1, **caractérisé en ce que** le moteur (36) est un moteur pas à pas.

3. Appareil de test de dissolution selon la revendication 1, **caractérisé en ce que** le moteur (36) est couplé via une courroie dentée (34) à deux colonnes télescopiques (30, 32) qui portent le cadre.
